# EUROPEAN PATENT APPLICATION

(11) **EP 4 807 349 A1**
(43) Date of publication of application: **16.09.2026**
(21) Application number: 26159376.8
(22) Date of filing: 18.02.2026
(51) Int. Cl.: G01N 21/898, B27L 1/00

(54) **METHOD FOR ADJUSTING THE OPERATION OF A DEBARKER**

(30) Priority: 13.03.2025 IT 202500005132
(71) Applicant: MICROTEC S.p.A., 39042 Bressanone (BZ) (IT)
(72) Inventor: GIUDICEANDREA, Federico, 39042 Bressanone BZ (IT)
(74) Representative: Ponchiroli, Simone

(57) **Abstract**

A method for adjusting the operation of a debarker which comprises an operating element (1) for removing bark (7) from a log (2) obtaining a debarked log (3) and operating according to adjustable operating parameters, comprising: a working step, using the operating element (1) in accordance with current operating parameters, in which the bark (7) is removed from the log (2); an analysis step, after the working, in which the debarked log (3) is analysed to obtain information about a real shape of the debarked log (3); a comparison step, after the analysis, in which the information about the real shape of the debarked log (3) is compared with information about an estimated shape of the debarked log (3); a set-up step, after the comparison, in which new operating parameters are set, or the current operating parameters are kept, for the operating element (1) based on the result of the comparison step.

## Description

This invention relates to a method for adjusting the operation of a debarker. In particular, this invention is applied for a debarker which comprises at least one operating element configured to remove the bark from a log to obtain a debarked log.

As is known, a debarker is an apparatus which works a log to debark it, that is to say, to remove the bark from the log.

In order to carry out debarking, on the market there are various types of debarkers, for example ring debarkers, milling head debarkers and drum debarkers. In general, the various types of debarkers are very different from each other. In fact, debarkers can be distinguished from each other in terms of operation, and as regards the components, the cost, etc.

Many prior art debarkers work in accordance with operating parameters which are fixed and cannot be changed in any way. Therefore, for these debarkers, it is not possible to change their operation based on the type of log to be worked. In other words, these debarkers work in the same way logs which are very different from each other and which have barks that have extremely different features, for example they have different thickness and different hardness.

In contrast, other debarkers have operating parameters which can be changed, where the operating parameters may be for example the pressure of the blade on the log and/or the feed speed of the log to the operating element of the debarker. In general, varying the operating parameters makes it possible to change the effect of the mechanical action applied by the operating element of the debarker on the log.

The operating parameters may be preset based on the type of log from which the bark must be removed. In general, in fact reference may be made to specific types of trees and the operating parameters may be predetermined based on features of a "standard" bark for logs of the specific type of tree. In more detail, given one type of tree (for example, oak, fir, etc.) from which the log was obtained, the predetermined operating parameters for working that specific type may be adopted with decidedly better results than can be obtained with debarkers in which the operating parameters are not adjustable.

However, these debarkers only partly solve the problems linked to debarkers in which the operating parameters are fixed and cannot be changed.

In fact, as can easily be inferred, non-optimal adjustment of the debarker may result in a mechanical action that is not very incisive, and therefore incomplete removal of the bark, or a mechanical action that is too incisive, and therefore removal not just of the bark but also of a quantity of underlying wood.

There may be many reasons why adjustment may not be optimal: not only may it be an initial error by an operator, but it is also possible that changed environmental or debarker conditions make the previously set operating parameters no longer high performance.

In this context the technical purpose which forms the basis of this invention is to provide a method for adjusting the operation of a debarker which at least partly overcome the above-mentioned disadvantages.

In particular the technical purpose of this invention is to provide a method for adjusting the operation of a debarker which allows the debarker to remove the bark more efficiently than prior art debarkers, also reducing any waste. The technical purpose specified and the aims indicated are substantially achieved by a method for adjusting the operation of a debarker as described in independent claim 1. Particular embodiments are described in the accompanying dependent claims.

Further features and the advantages of this invention will be more apparent in the detailed description of several preferred, non-limiting embodiments, of a method for adjusting the operation of a debarker illustrated in the accompanying drawings, in which:
- Figure 1 schematically shows an embodiment of a method for adjusting the operation of a debarker in accordance with this invention; and
- Figure 2 schematically shows a comparison step which is part of the method shown in Figure 1.

This invention relates to a method for adjusting the operation of a debarker, several steps of which are advantageously implemented by means of a computer. In particular, this invention relates to a method for adjusting the operation of a debarker which comprises at least one operating element 1 which is configured to remove bark 7 from a log 2 to thereby obtain a debarked log 3. The operating element 1 operates in accordance with operating parameters which are adjustable. It should be emphasised that this method is applied for debarkers which comprise an operating element 1, or multiple operating elements 1, of any type provided that the operating parameters can be adjusted. For example, in some embodiments it is possible that the operating element 1 comprises a plurality of knives. In this way, the operating parameters may advantageously comprise, for example, the feed speed of the log 2 and/or the speed of movement of the knives and/or the pressure of the knives on the log 2. In any case, it should be emphasised that both the possible types of debarkers, and the possible types of operating elements 1 of the debarkers, are known to a person expert in the sector, and for this reason they will not be described in further detail below.

The method according to this invention comprises first a working step which is carried out using the operating element 1 of the debarker in accordance with current operating parameters (that is to say, making the debarker work with the current operating parameters). During the working step the bark 7 is removed from the log 2 in such a way as to obtain the debarked log 3. As previously described, since the types of operating elements 1 which may be part of the debarker are known, the ways in which the working step may be carried out are also known to a person expert in the sector.

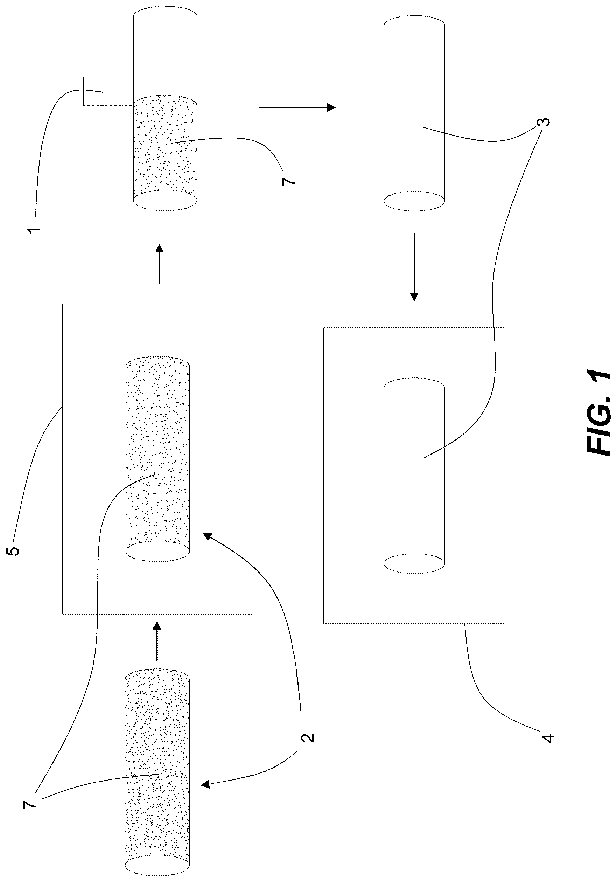

Second, the method comprises an analysis step, which is carried out after the working step. In the analysis step, the debarked log 3 is analysed with an electronic apparatus 4 (schematically shown as a rectangle in Figure 1) to obtain information about a real shape of the debarked log 3. In the context of this invention, the expression "real shape" of the debarked log 3 means the shape of the debarked log 3 which was effectively obtained by carrying out the working step.

The analysis step may be carried out in various ways.

In some embodiments, in the analysis step at least one tomography scan of the debarked log 3 is carried out. From the tomography scan at least one three-dimensional tomography image of the debarked log 3 is obtained. In these embodiments, advantageously the information about the real shape of the debarked log 3 therefore comprises the three-dimensional tomography image of the debarked log 3 and a digital three-dimensional model of it (and, at least implicitly, all of the measurements obtainable from them). Preferably, the three-dimensional tomography image and the digital three-dimensional model are saved in such a way that they are available saved and can be used in subsequent steps of the method such as, for example, in the comparison step, as will become apparent from the following description.

In other embodiments, in the analysis step at least one three-dimensional scan of the outer surface of the debarked log 3 is carried out, for example by means of a laser triangulation apparatus, in this case too obtaining a digital three-dimensional model of the debarked log 3. In these embodiments too, the information about the real shape of the debarked log 3 comprises the digital three-dimensional model of it (and, at least implicitly, all of the measurements obtainable from it). Similarly to what was previously described, preferably the digital three-dimensional model is saved in such a way that it is available saved and can be used in subsequent steps of the method.

The ways of carrying out the analysis step described above are the preferred ways, but should not be understood to be limiting for this invention. In fact, it is possible for the analysis step to be carried out in other ways which allow information about the real shape of the debarked log 3 to be obtained.

In accordance with an innovative aspect of this invention, the method also comprises both a comparison step, and a set-up step.

In the comparison step, which is carried out after the analysis step, a computer 6 is used to compare the information about the real shape of the debarked log 3, with information about an estimated shape of the debarked log 3. In the context of this invention, the expression "estimated shape" of the debarked log 3 means a target shape of the debarked log 3 determined before the debarking operation and which, ideally, may be considered the shape which the debarked log 3 should have as a result of carrying out the working step, if the operating element 1 of the debarker were to remove only and completely the bark 7 (that is to say, without wasting any wood and without leaving residual bark 7 on the log 2).

With reference to the comparison step, in some embodiments the information about the estimated shape of the debarked log 3 is available saved, whilst in other embodiments the method involves carrying out an estimation step, before the working step, during which the information about the estimated shape of the debarked log 3 is determined.

In the embodiments in which the information about the estimated shape of the debarked log 3 is available saved, that information is advantageously obtained previously, for example by analysing the log 2 in ways similar to those described below with reference to the estimation step.

In contrast, in the embodiments involving the estimation step, the method comprises that step, which is carried out before the working step. Advantageously, in the estimation step the information about the estimated shape of the debarked log 3 is estimated starting from a non-destructive analysis of the log 2 including the bark 7.

In some embodiments, in the estimation step at least one tomography scan of the log 2 including the bark 7 is carried out (using a tomography scanner 5 schematically shown as a rectangle in Figure 1), in such a way as to obtain either at least one three-dimensional tomography image of the log 2 including the bark 7, or a digital three-dimensional tomography model of the log 2 including the bark 7 (or both the three-dimensional tomography images of the log 2 including the bark 7, and the digital three-dimensional tomography model of the log 2 including the bark 7). In this case, the information about the estimated shape of the debarked log 3 is obtained respectively from the three-dimensional tomography image of the log 2 including the bark 7 and/or from the digital three-dimensional tomography model of the log 2 itself including the bark 7, from which the bark 7 is virtually removed, advantageously using algorithms for processing three-dimensional images.

In others amongst these embodiments, in contrast, in the estimation step the outer shape of the log 2 including the bark 7 is measured, advantageously by carrying out at least one scan of the three-dimensional shape of the log 2 to obtain a digital three-dimensional model of the log 2 including the bark 7, the thickness of the bark 7 is estimated preferably with known techniques, for example by taking one or more X-rays of the log 2 including the bark 7, and the information about the estimated shape of the debarked log 3 is determined by virtually removing the estimated thickness of the bark 7 from the measured outer shape of the log 2 including the bark 7 (that is to say, by virtually removing the estimated thickness of the bark 7 from the digital three-dimensional model of the log 2 including the bark 7). In this case, when to estimate the thickness of the bark 7 the one or more X-rays of the log 2 including the bark 7 are taken, one or more two-dimensional X-ray images of the log 2 including the bark 7 are obtained and those one or more two-dimensional X-ray images are examined to estimate the thickness of the bark 7 (with techniques which are known in themselves advantageously applied at the edges of the X-ray images).

Alternatively to measuring the outer shape of the log 2 including the bark 7, in other embodiments it is possible to obtain the outer shape of the log 2 including the bark 7 which was previously saved, if that outer shape is advantageously present having been saved. Similarly to what was previously described, in this case too the thickness of the bark 7 is estimated (if it was not already previously estimated) and the information about the estimated shape of the debarked log 3 is determined by virtually removing, from the outer shape of the log 2 including the bark 7 the estimated thickness of the bark 7.

In other embodiments it is possible that in the estimation step, instead of taking the one or more X-rays of the log 2 including the bark 7, the thickness of the bark 7 is estimated using at least one calculation algorithm. In fact, in order to calculate the thickness of the bark 7, known mathematical formulae, of empirical origin, may advantageously be used, which based on known parameters, such as for example the type of wood and the diameter of the log 2, allow the thickness of the bark 7 to be calculated with optimum precision.

In other embodiments, in the estimation step data about the log 2 including the bark 7 are processed using a deep learning algorithm to obtain information about the estimated shape of the debarked log 3. For example, in order to obtain that data about the log 2 including the bark 7 at least one, preferably more than one, of the following actions may be carried out: taking a three-dimensional measurement of the log 2 including the bark 7 and/or obtaining at least one colour image of the log 2 including the bark 7 and/or taking an X-ray of the log 2 including the bark 7 and/or carrying out a tomography scan of the log 2 including the bark 7.

In order to obtain the training data for that deep learning algorithm it is sufficient to acquire the data of interest with reference to a plurality of logs 2 including the bark, to precisely debark them (even with manual intervention by operators where necessary) and then to precisely measure all of the debarked logs 3 obtained in this way.

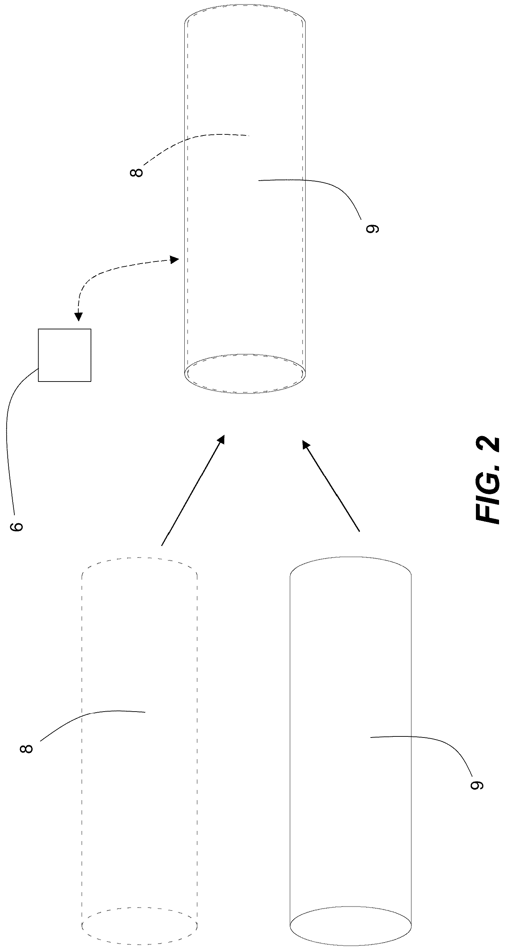

Advantageously, the information about the real shape of the debarked log 3 and the information about the estimated shape of the debarked log 3 is information of the same type. Preferably, in this case it is not necessary to carry out processing of the information in order to convert it from one type to another. For example, if both the information about the real shape of the debarked log 3 and the information about the estimated shape of the debarked log 3 is three-dimensional tomography images obtained using a tomography scanner, the comparison step may be carried out by comparing the three-dimensional tomography images. Advantageously, the comparison of the three-dimensional images is carried out using known software for processing three-dimensional images. An example of a comparison step carried out in these ways is shown for example in Figure 2, in which broken lines are used to show the estimated shape 8 of the debarked log 3 and continuous lines are used to show the real shape 9 of the debarked log 3. Moreover, in the case of Figure 2, it can be seen how the real shape 9 does not correspond to the estimated shape 8.

In the set-up step, which is carried out after the comparison step, based on the result of the comparison step new operating parameters are set for the operating element 1 or the current operating parameters are kept for the operating element 1. If the new operating parameters are set, advantageously they may be set automatically by a computer connected to the debarker. However, the new operating parameters may still be set manually by an operator.

In the preferred embodiments, the set-up step is based on a debarking error which is obtained by carrying out the comparison step. In particular, during the comparison step the debarking error is determined which is obtained from the comparison between the information about the real shape of the debarked log 3 and the information about the estimated shape of the debarked log 3. In Figure 2, the debarking error can be identified in the right-hand part in the deviation between the real shape 9 and the estimated shape 8 which are superposed. Preferably, based on the debarking error determined in the comparison step, during the set-up step the new operating parameters are set for the operating element 1, or the current operating parameters are kept for the operating element 1.

In the embodiments in which the debarking error is determined, the comparison step also comprises, advantageously, an assessment sub-step in which the debarking error is compared with a predetermined error threshold. In these embodiments, in the set-up step the choice to set the new operating parameters or to keep the current operating parameters, is made based on the comparison between the debarking error and the predetermined error threshold which is carried out during the assessment sub-step. In particular, when the debarking error is greater than the predetermined error threshold, advantageously the new operating parameters are set. In contrast, when the debarking error is less than the predetermined error threshold, advantageously the current operating parameters are kept.

In the preferred embodiments, the method also comprises an inspection step, which is carried out after the working step and before the set-up step. In the inspection step the debarked log 3 is inspected, to obtain information about the presence of any residual bark 7. In these embodiments, during the set-up step the new operating parameters are set for the operating element 1, or the current operating parameters are kept for the operating element 1, based on the result of both the comparison step and the inspection step. For example, if during the inspection step the presence of residual bark 7 is detected at a zone of the log 2 in which that residual bark 7 should not be present, advantageously new operating parameters may be set which are selected in such a way as to remove more bark 7 than is removed using the current operating parameters. Preferably, the inspection step is carried out either manually by an operator or automatically by using an electronic system for image acquisition and processing.

In the context of its use at industrial level, the method according to this invention is advantageously applied for debarkers which work many logs 2 one after another, in such a way as to obtain corresponding debarked logs 3. Each debarked log 3 is therefore advantageously obtained from the working, and in particular from the debarking, of a log 2 which is part of a starting plurality of logs 2.

In these embodiments, the working step, the analysis step and the comparison step are preferably carried out on each log 2, and the results of the comparison steps are saved. Alternatively, the working step, the analysis step and the comparison step may be carried out only on a plurality of logs 2 which corresponds to a sub-group of all of the logs 2 worked by the plant. In this case too, the results of the comparison steps are saved.

In these embodiments in which the results of the comparison steps carried out for a plurality of different logs are saved, the set-up step may be carried out in a way that is different from that described above. In particular, it may be the case that, during the set-up step, the new operating parameters are set for the operating element 1, or the current operating parameters are kept for the operating element 1, by considering as a whole the results of the comparison steps which are carried out on the logs 2.

Preferably, in each comparison step a relative debarking error is determined, obtained from the comparison between the information about the real shape and the information about the estimated shape of the respective debarked log 3.

Once all of the debarking errors determined for a predetermined group of logs 2 are available, in some cases, an average debarking error may also be calculated and the set-up step carried out using that average debarking error. For example, the new operating parameters may be set for the operating element 1, or the current operating parameters may be kept for the operating element 1, based on the average debarking error. Alternatively, instead of an average debarking error a median debarking error may be calculated and used. In other cases, in contrast, instead of an average debarking error it is possible to calculate and use a working debarking error calculated based on statistical type rules (for example, taking into consideration the standard deviation of every single measurement).

In some cases, it is possible to associate with each debarking error obtained a weight which indicates a higher or lower importance of the debarking error with which the weight is associated. For example, if for a same debarker bark thickness assessments were available which were carried out in different ways, some starting from tomography scan data, others using simple empirical calculation algorithms, preferably it would be possible to associate with the debarking errors obtained from the comparison between three-dimensional tomography images weights which indicate a higher importance than weights which could be associated with the debarking errors obtained from the comparison by using the shape under the bark of the log 2 based on the calculation of the thickness of the bark 7. In fact, the result of the tomography scan is generally more reliable than the result of the calculation of the thickness of the bark 7.

However, more often, all of the estimates of the shape under the bark are carried out in one way; in these cases, the different weights may advantageously be applied based on the outer features of the log 2. For example, if the outer surface of the log 2 is smooth and regular, the corresponding debarking error may be assigned a weight which indicates a high degree of reliability. Otherwise, if the surface of the log is irregular and has hollows, bumps or other defects which may impair the correct removal of the bark 7 even with optimal debarker set-up, the corresponding debarking error may be assigned a weight which indicates a low degree of reliability.

In order to be able to correctly carry out the method described up to now it is necessary to know from which log 2 with bark each debarked log 3 was obtained. In some embodiments that is easily achieved by following or tracking the movements of the log between the zones in which the various working operations are carried out (for example with photocells and/or encoders). In other embodiments, in the known way an identifying element (a text, a bar code, etc.) may be applied to each log 2, allowing it to be recognised at any time; advantageously that identifying element may be applied to the front face.

When the identity of the debarked log 3 is unknown it is therefore necessary to carry out an identification which, advantageously, in some preferred embodiments of the method also comprises both an examination step, and a recognition step.

In the examination step, which is carried out after the working step, the debarked log 3 which was obtained from the working step is studied to identify one or more identifying features of that debarked log 3. In contrast, in the recognition step, which is carried out after the examination step, the log 2 from which the debarked log 3 was obtained is recognised. The recognition step is carried out by comparing the one or more identifying features of the debarked log 3 with one or more identifying features of each starting log 2, which were previously saved. In the context of this invention, the expression "identifying features" means the set of unique biometric features of the log 2 and of the debarked log 3 such as, for example, the distribution of the knots in the log 2 and in the debarked log 3. As is known, the set of unique biometric features (that is to say, identifying features) of the log 2 is also called the "fingerprint".

As regards the identifying features of the logs 2 including the bark 7, in some embodiments the identifying features of the logs 2 are already available saved (for example because they were already previously identified), whilst in other embodiments the identifying features of the logs 2 are not available saved. In the former embodiments, it is for example possible that the log 2 was scanned immediately after cutting using a tomography scanner (it is possible that in this step the information about the estimated shape of the debarked log 3 and the one or more identifying features of the log 2 are obtained simultaneously). In contrast, in the latter embodiments, the method preferably comprises a further step of preliminary examination, which is carried out before the working step (advantageously in combination with the estimation step), during which the logs 2 including the bark 7 are studied, to identify the one or more identifying features of those logs 2.

The examination step and the recognition step, and if necessary the further preliminary examination step, allow each debarked log 3 to be associated with the log 2 including the bark 7 from which that debarked log 3 was obtained. That is advantageous if the debarker operates on many logs 2 one after another whose movements are not tracked in another way.

This invention brings important advantages.

In fact, thanks to this invention, it was possible to develop a method for adjusting the operation of a debarker which allows bark to be removed more efficiently than by prior art debarkers, also reducing any waste.

Finally, it should be noticed that this invention is relatively easy to produce and that even the cost linked to implementing the invention is not very high. The invention described above may be modified and adapted in several ways without thereby departing from the scope of the inventive concept.

All details may be substituted with other technically equivalent elements and the materials used, as well as the shapes and dimensions of the various components, may vary according to requirements.

## Claims

1. A method for adjusting the operation of a debarker which comprises at least one operating element (1) which is configured to remove bark (7) from a log (2) to obtain a debarked log (3) and which operates in accordance with operating parameters which are adjustable,
the method comprising the following steps:
a working step which is carried out using the at least one operating element (1) of the debarker in accordance with current operating parameters, during which the bark (7) is removed from the log (2) to obtain the debarked log (3);
an analysis step which is carried out after the working step, during which an electronic apparatus (4) is used to analyse the debarked log (3) to obtain information about a real shape of the debarked log (3);
a comparison step which is carried out after the analysis step, during which a computer (6) is used to compare the information about the real shape of the debarked log (3) with information about an estimated shape of the debarked log (3); and
a set-up step which is carried out after the comparison step, during which new operating parameters are set for the at least one operating element (1), or the current operating parameters are kept for the at least one operating element (1), based on the result of the comparison step.

2. The method according to claim 1, wherein:
in the comparison step a debarking error is determined which is obtained from the comparison between the information about the real shape of the debarked log (3) and the information about the estimated shape of the debarked log (3); and
in the set-up step the new operating parameters are set for the at least one operating element (1), or the current operating parameters are kept for the at least one operating element (1), based on the debarking error determined in the comparison step.

3. The method according to claim 2, wherein the comparison step also comprises an assessment sub-step in which the debarking error is compared with a predetermined error threshold, and wherein in the set-up step alternatively:
when the debarking error is greater than the predetermined error threshold, the new operating parameters are set; and
when the debarking error is less than the predetermined error threshold, the current operating parameters are kept.

4. The method according to any one of claims 1 to 3, wherein in the analysis step at least one digital three-dimensional model of the debarked log (3) is obtained, the information about the real shape of the debarked log (3) comprising said digital three-dimensional model of the debarked log (3).

5. The method according to claim 4, wherein in the analysis step at least one surface three-dimensional scan of the debarked log (3) is carried out to obtain the digital three-dimensional model of the debarked log (3).

6. The method according to any one of claims 1 to 5, wherein the information about the estimated shape of the debarked log (3) is available saved.

7. The method according to any one of claims 1 to 5, wherein the method also comprises an estimation step, which is carried out before the working step, in which the information about the estimated shape of the debarked log (3) is determined.

8. The method according to claim 7, wherein in the estimation step at least one tomography scan of the log (2) including the bark (7) is carried out, to obtain at least one three-dimensional tomography image of the log (2) including the bark (7) and/or a digital three-dimensional tomography model of the log (2) including the bark (7), the information about the estimated shape of the debarked log (3) being obtained respectively from said at least one three-dimensional tomography image of the log (2) including the bark (7) and/or from the digital three-dimensional tomography model of the log (2) including the bark (7), by virtually removing the bark (7).

9. The method according to claim 7, wherein in the estimation step either an outer shape of the log (2) including the bark (7) is measured or a saved outer shape of the log (2) including the bark (7) is obtained, a thickness of the bark (7) is estimated, and the information about the estimated shape of the debarked log (3) is determined by virtually removing the estimated thickness of the bark (7) from the outer shape of the log (2) including the bark (7).

10. The method according to claim 9, wherein in the estimation step one or more X-rays of the log (2) including the bark (7) are taken to obtain one or more two-dimensional X-ray images of the log (2) including the bark (7), and said one or more two-dimensional X-ray images are examined to estimate the thickness of the bark (7).

11. The method according to claim 9, wherein in the estimation step the thickness of the bark (7) is estimated using at least one calculation algorithm.

12. The method according to claim 7, wherein in the estimation step at least one of the following actions is carried out, to obtain data about the log (2) including the bark (7): a three-dimensional measurement of the log (2) including the bark (7) is taken; at least one colour image of the log (2) including the bark (7) is obtained; an X-ray of the log (2) including the bark (7) is taken; a tomography scan of the log (2) including the bark (7) is carried out;
and wherein said data about the log (2) including the bark (7) are processed using a deep learning algorithm to obtain information about the estimated shape of the debarked log (3).

13. The method according to any one of claims 1 to 12, also comprising an inspection step, which is carried out after the working step and before the set-up step, in which the debarked log (3) is inspected to obtain information about the presence of any residual bark (7),
and wherein in the set-up step the new operating parameters are set for the at least one operating element (1), or the current operating parameters are kept for the at least one operating element (1), also based on the result of the inspection step.

14. The method according to any one of claims 1 to 13, also comprising:
an examination step, which is carried out after the working step, in which the debarked log (3) which was obtained from the working step is studied to identify one or more identifying features of said debarked log (3); and
a recognition step, which is carried out after the examination step, in which a log (2) of a plurality of logs (2) from which said debarked log (3) was obtained is recognised,
the recognition step being carried out by comparing the one or more identifying features of the debarked log (3) with one or more identifying features of each log (2) of said plurality of logs (2) which were previously saved.

15. The method according to any one of claims 1 to 14, wherein the working step, the analysis step and the comparison step are carried out on each log (2) of a plurality of logs (2), the results of the comparison steps being saved, and wherein in the set-up step the new operating parameters are set for the at least one operating element (1), or the current operating parameters are kept for the at least one operating element (1), by considering as a whole the results of the comparison steps carried out on the plurality of logs (2).
